# EUROPEAN PATENT SPECIFICATION

(11) **EP 2 393 485 B1**
(45) Date of publication and mention of the grant of the patent: **29.07.2015**
(21) Application number: 10703766.5
(22) Date of filing: 04.02.2010
(51) Int. Cl.: A61K 9/20, A61K 9/24, A61K 9/50, A61K 47/24, A61K 31/47, A61K 31/513, A61K 31/522, A61K 31/5377, A61P 31/18

(54) **BILAYER TABLETS COMPRISING ELVITEGRAVIR, COBICISTAT, EMTRICITABINE AND TENOFOVIR**
ZWEISCHICHTIGE TABLETTE ENTHALTEND ELVITEGRAVIR, COBICISTAT, EMTRICITABINE UND TENOFOVIR
COMPRIMÉ BICOUCHE CONTENANT DE L'ELVITEGRAVIR, DU COBICISTAT, DE L'EMTRICITABINE ET DU TENOFOVIR

(30) Priority: 06.02.2009 US 150652 P; 06.02.2009 US 150655 P
(43) Date of publication of application: 14.12.2011
(73) Proprietor: Gilead Sciences, Inc., Foster City, CA 94404 (US)
(72) Inventor: OLIYAI, Reza, Foster City, California 94404 (US); MENNING, Mark, M., Foster City, California 94404 (US); KOZIARA, Joanna, M., Foster City, California 94404 (US)
(74) Representative: Hallybone, Huw George
(86) International application number: PCT/US2010/023226
(87) International publication number: WO 2010/091197

(56) References cited:
- WO-A1-2008/103949
- US-B2- 7 176 220
- L. SZCZECH: "Tenofovir nephrotoxicity: focusing research questions and putting them into clinical context", THE JOURNAL OF INFECTIOUS DISEASES, vol. 197, no. 1, 2008, pages 7-9, XP002630507,
- FOR THE UK COLLABORATIVE HIV COHORT (CHIC) STUDY PHILLIPS ET AL: "Risk of extensive virological failure to the three original antiretroviral drug classes over long-term follow-up from the start of therapy in patients with HIV infection: an observational cohort study", LANCET THE, LANCET LIMITED. LONDON, GB, vol. 370, no. 9603, 6 December 2007 (2007-12-06), pages 1923-1928, XP022379072, ISSN: 0140-6736
- V. VON WYL ET AL.: "Emergence of HIV-1 drug resistance in previously untreated patients initiating combination antiretroviral treatment", ARCHIVES OF INTERNAL MEDICINE, vol. 167, no. 16, 2007, pages 1782-1790, XP002630508,
- S. HAMMER ET AL.: "Antiretroviral treatment of adult HIV infection. 2008 Recommendations of the International AIDS Society- USA panel.", JAMA, THE JOURNAL OF THE AMERICAN MEDICAL ASSOCIATION, vol. 300, no. 5, 2008, pages 555-570, XP8134902,
- J. MARTINEZ-CAJAS ET AL.: "Antiretroviral therapy- Optimal sequencing of therapy to avoid resistance", DRUGS, vol. 68, no. 1, 2008, pages 43-72, XP8134919,
- GERMAN P ET AL: "pharmacokinetics and bioavailability of an integrase and novel pharmacoenhancer-containing single tablet fixed dose combination", JOURNAL OF ACQUIRED IMMUNE DEFICIENCY SYNDROMES, LIPPINCOTT WILLIAMS AND WILKINS,PHILADELPHIA, PA, US, vol. 55, no. 3, 1 November 2010 (2010-11-01), pages 323-329, XP008134624, ISSN: 1525-4135
- "Full Prescribing Information Viread (TDF)", 1 January 2012 (2012-01-01), Gilead Sciences, Inc, Foster City, CA 94404, USA
- "Full Prescribing Information Emtriva (FTC)", 1 January 2012 (2012-01-01), Gilad Sciences, Inc, Foster City, CA 94404, USA

## Description

### Background

US 7,176,220 discloses compounds that are reported to be useful as integrase inhibitors. One particular compound is the compound of Formula I (i.e. elvitegravir).

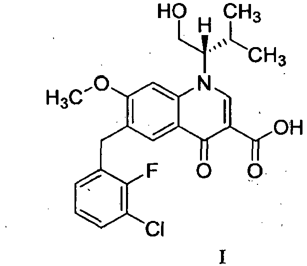

WO 2005/113508 provides certain specific crystalline forms of the compound of Formula I. The specific crystalline forms are reported to have superior physical and chemical stability compared to other physical forms of the compound.

WO 2008/010921 describes compounds that improve the pharmacokinetics of a co-administered drug by inhibiting cytochrome P450 monooxygenase. One such inhibitor is the compound of Formula II.

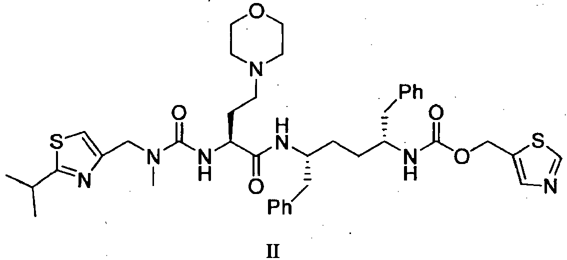

WO 2008/010921 also describes pharmaceutical compositions comprising the compounds and provides for compositions that include at least one additional therapeutic agent. On pages 170 and 174 WO 2008/010921 further describes combinations of the disclosed compounds (e.g. a compound of Formula II). Included among the listing of different agents is the compound of Formula I (i.e. elvitegravir), the compound of Formula III (i.e. emtricitabine) and the salt of Formula IV (i.e. tenofovir disoproxil fumarate). A discussion regarding formulations of the compounds can also be found at pages 159-168.

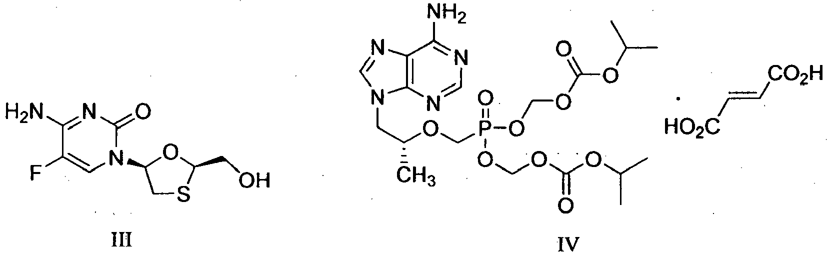

WO 2008/103949 also discloses compounds that are reported to be useful to modify the pharmacokinetics of a co-administered drug by inhibiting cytochrome P450 monooxygenase. Page 199 of WO 2008/103949 describes the specific combination of the compounds of Formula I, Formula II, Formula III and Formula IV. Formulations are discussed on pages 181-190.

Despite these reports there currently is a need for improved solid dose forms (e.g. tablets) for the delivery of multi-agent compositions. There is also a need for improved solid dose forms comprising the compounds of Formulas I, II, III and IV that have enhanced or acceptable chemical stability, physical stability, release characteristics, or dosage uniformity. There is also a need for improved processes to prepare solid dose forms of compositions comprising the compounds of Formulas I, II, III and IV.

### Summary of the Invention

The present invention provides solid dose forms (e.g. tablets) comprising the compounds of Formula I, Formula II, Formula III and the salt of Formula IV and methods to prepare the solid dose forms. Applicant has discovered that tablets prepared in a bilayer manner have superior properties to alternatively structured tablets. This discovery represents an advance in the development of combination therapy for the treatment of viral infections such as HIV.

Accordingly, in one embodiment the invention provides a tablet comprising a first layer and a second layer wherein;
a) the first layer comprises:
   a compound of Formula I: 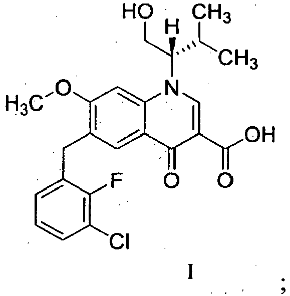 and
   a compound of Formula II: 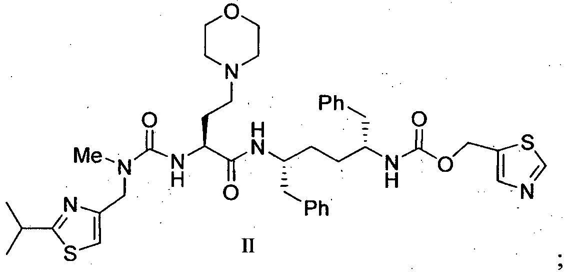 and optionally a pharmaceutically acceptable carrier;
b) the second layer comprises:
   a compound of Formula III: 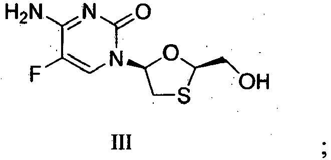 and
   a salt of Formula IV: 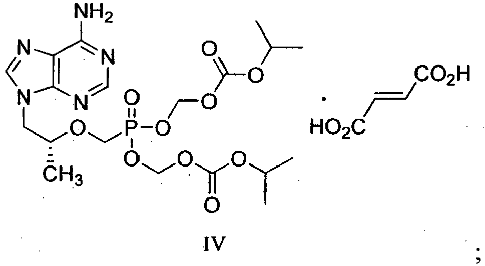
   and optionally a pharmaceutically acceptable carrier.

In one embodiment the invention provides a pharmaceutical composition that comprises 150 mg ± 10% of the compound of Formula I; 150 mg ± 10% of the compound of Formula II; 200 mg ± 10% of the compound of Formula III; and 300 mg ± 10% of the compound of Formula IV.

In one embodiment the invention provides a pharmaceutical composition that comprises 150 mg ± 5% of the compound of Formula I; 150 mg ± 5% of the compound of Formula II; 200 mg ± 5% of the compound of Formula III; and 300 mg ± 5% of the compound of Formula IV.

In one embodiment the invention provides a pharmaceutical composition that comprises 150 mg ± 2% of the compound of Formula I; 150 mg ± 2% of the compound of Formula II; 200 mg ± 2% of the compound of Formula III; and 300 mg ± 2% of the compound of Formula IV.

In one embodiment the invention provides a pharmaceutical composition that consists of 150 mg ± 5% of the compound of Formula I; 150 mg ± 5% of the compound of Formula II; 200 mg ± 5% of the compound of Formula III; and 300 mg ± 5% of the compound of Formula IV as pharmaceutically active ingredients; and one or more pharmaceutically acceptable carriers.

In one embodiment the invention provides a pharmaceutical composition that consists of 150 mg ± 2% of the compound of Formula I; 150 mg ± 2% of the compound of Formula II; 200 mg ± 2% of the compound of Formula III; and 300 mg ± 2% of the compound of Formula IV as pharmaceutically active ingredients; and one or more pharmaceutically acceptable carriers.

In one embodiment the invention provides a tablet that consists of the following:

| | **Compound of Formula II 150 mg Formulation** | |
|---|---|---|
| **Components** | **% w/w** | **mg/tablet** |
| Compound of Formula III | 13.9 | 200.0 |
| Salt of Formula IV | 20.9 | 300.0 |
| Compound of Formula I | 10.4 | 150.0 |
| Compound of Formula II | 10.4 | 150.0 |
| Colloidal Silicon Dioxide | 12.0 | 172.5 |
| Lactose Monohydrate | 0.8 | 10.9 |
| Microcrystalline Cellulose | 20.8 | 299.5 |
| Hydroxypropyl Cellulose | 0.5 | 7.5 |
| Hydroxypropyl Cellulose | 0.6 | 9.0 |
| Sodium Lauryl Sulfate | 0.8 | 11.3 |
| Croscarmellose Sodium | 7.3 | 104.3 |
| Magnesium Stearate | 1.6 | 22.4 |
| Total | 100 | 1437. |

The invention also provides methods for preparing tablets of the invention that are described herein, as well as intermediate compositions and articles that are useful for preparing compositions of the invention. Accordingly, in one embodiment the invention provides a method for preparing a tablet of the invention comprising: compressing a composition that comprises the compound of formula I and the compound of formula II to provide a first pressed layer; adding the compound of formula III and the salt of formula IV to the first pressed layer; and compressing to provide the tablet of the invention. In one embodiment of the invention, the composition that comprises the compound of formula I and the compound of formula II is compressed to provide a soft layer; a mixture that comprises the compound of formula III and the salt of formula IV is added to the soft layer to provide a final combination; and the final combination is compressed to provide the tablet of the invention. In one embodiment, the methods of the invention can further comprise coating the tablet.

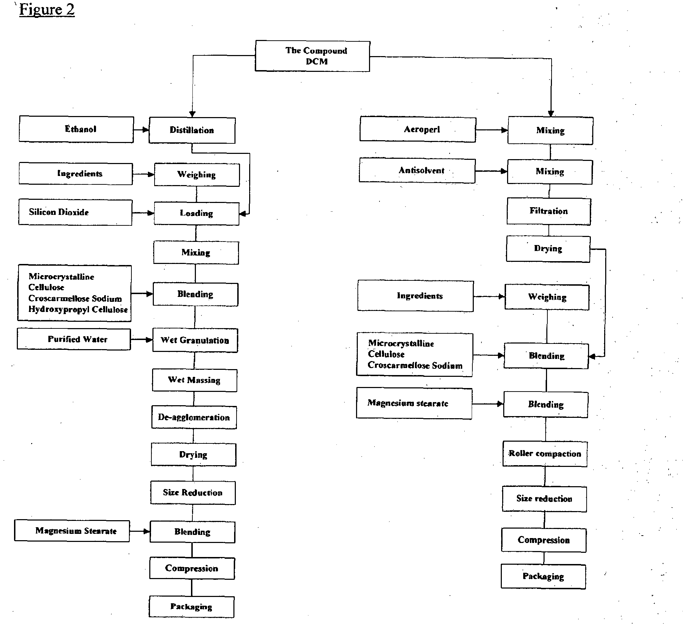

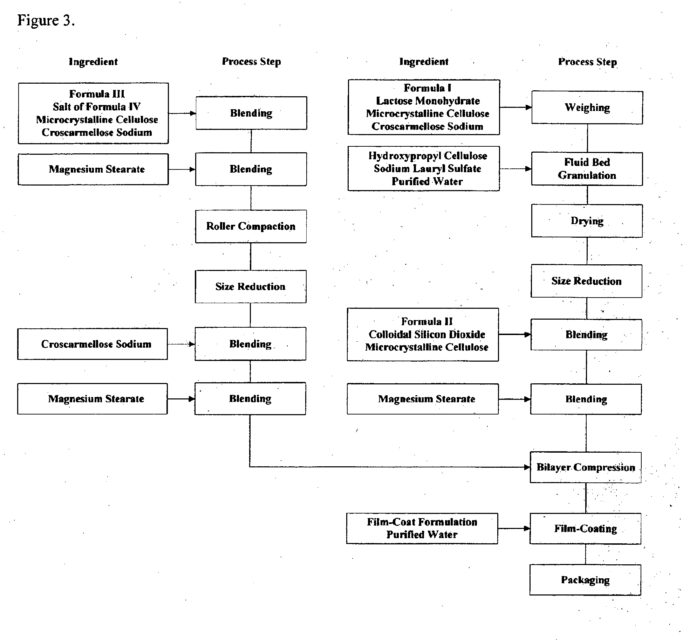

The invention also provides the methods illustrated in Figures 2 and 3 and in the Examples herein, which are useful for preparing intermediate compositions and tablets of the invention.

### Brief Description of the Figures

FIG. 1. Illustrates a tablet of the invention.
FIG. 2. Illustrates the preparation of compositions of the invention.
FIG. 3. Illustrates the preparation of compositions of the invention.

### Detailed Description

Specific values listed below for ranges and terms are for illustration only; they do not exclude other values.

### Compound Amounts and Compound Weight Ratios

The tablets of the invention comprise compounds of Formula I, Formula II, Formula III and the salt of Formula IV. The weights and ratios described herein relate to the combination therapy as described. It is to be understood that the amounts of the various compounds can vary while remaining within the scope of the invention. In one embodiment the amount of the compound of Formula I in a tablet of the invention is 150 mg ± 10%. In another embodiment the amount of the compound of Formula I in a tablet of the invention is 150 mg ± 5%. In another embodiment the amount of the compound of Formula I in a tablet of the invention is 150 mg ± 2%. In one embodiment the amount of the compound of Formula II in a tablet of the invention is 150 mg ± 10%. In another embodiment the amount of the compound of Formula II in a tablet of the invention is 150 mg ± 5%. In another embodiment the amount of the compound of Formula II in a tablet of the invention is 150 mg ± 2%. In one embodiment of the amount of the compound of Formula III in a tablet of the invention is 200 mg ± 10%. In another embodiment the amount of the compound of Formula III in a tablet of the invention is 200 mg ± 5%. In another embodiment the amount of the compound of Formula III in a tablet of the invention is 200 mg ± 2%. In one embodiment the amount of the salt of Formula IV in a tablet of the invention is 300 mg ±10%. In another embodiment the amount of the salt of Formula IV in a tablet of the invention is 300 mg ± 5%. In another embodiment the amount of the salt of Formula IV in a tablet of the invention is 300 mg ± 2%.

It is also to be understood that various weight ratios of the compounds to one another within the combination therapy as described can vary while remaining within the scope of the invention. In one embodiment the weight ratio of the compound of Formula I to the compound of Formula II in a tablet of the invention is 1 ± 0.8. In another embodiment the weight ratio of the compound of Formula I to the compound of Formula II in a tablet of the invention is 1 ± 0.5. In another embodiment the weight ratio of the compound of Formula I to the compound of Formula II in a tablet of the invention is 1 ± 0.3. In another embodiment the weight ratio of the compound of Formula I to the compound of Formula II in a tablet of the invention is 1 ± 0.1.

### Pharmaceutical Formulations

The pharmaceutical compositions of the invention may be formulated with conventional carriers. While it is possible for the active ingredients of the composition to be administered alone it may be preferable to present them as pharmaceutical formulations. As described herein the pharmaceutical formulations of the invention may comprise compounds of Formulas I, II, III and IV together with one or more acceptable carriers. The carrier(s) should be "acceptable" in the sense of being compatible with the other ingredients of the formulation and physiologically innocuous to the recipient thereof. Accordingly, in one embodiment, the application provides for pharmaceutical compositions comprising the compounds of Formulas I, II, III and IV and a pharmaceutically acceptable carrier

As used herein the term carrier includes excipients, glidants, fillers, binders, lubricant, diluents, preservatives, surface active agents, dispersing agents and the like. The term carrier also includes agents such sweetening agents, flavoring agents, coloring agents and preserving agents. Furthermore, these terms include the values mentioned herein as well as values in accord with ordinary practice. Tablets will generally contain excipients, glidants, fillers and binders. All formulations can optionally contain excipients such as those set forth in the Handbook of Pharmaceutical Excipients (APhA Publications, Washington, DC). Excipients include ascorbic acid and other antioxidants, chelating agents such as EDTA, carbohydrates such as dextrin, hydroxyalkylcellulose, hydroxyalkylmethylcellulose and stearic acid. The pH of the formulations ranges from 3 to 11, but is ordinarily 7 to 10.

The pharmaceutical formulations include those suitable for the administration routes discussed herein. The pharmaceutical formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient(s) with the carrier which constitutes one or more accessory ingredients. In general the formulations can be prepared by uniformly and intimately bringing into association the active ingredient(s) with liquid carriers or finely divided solid carriers or both, and then, if necessary, shaping the product (e.g. as a unit dosage form).

A particular carrier that can be used in combination with the compound of Formula II is a silica particle. Typically, silica particles comprise a granular hydrophilic fumed silica that has a mean particle diameter of 10 to 120 micron and a BET surface area of 40 to 400 m²/g (determined according to DIN 66 131 with nitrogen). The silica particles also typically have a pore volume of 0.5 to 2.5 mL/g, wherein less than about 5% of the overall pore volume has a pore diameter of less than 5 nm, the remainder being mesopores and macropores. Additionally, the silica particles typically have a pH in the range of 3.6 to 8.5 and a tapped density of 220 to 700 g/L.

A specific silica material (particle) that is particularly useful in the compositions of the invention is AEROPERL® 300 (fumed silica), which is available from Evonik Degussa AG, Dusseldorf, Germany. However, other materials having physical and chemical properties similar to the silica materials described herein can also be used, e.g. calcium silicate (such as Zeopharm), or magnesium aluminometasilicate (such as Neusilin). Silica particles that have a mean grain diameter of 10 to 120 micron are useful. Silica particles that have a mean grain diameter of 20-40 micron are also useful. Silica particles that have a BET surface area of 40 to 400 m²/g are useful. Silica particles that have a BET surface area of at least 150 m²/g, or at least 200 m²/g, or at least 250 m²/g or at least 275 m²/g are also useful.

In one embodiment, the compound of Formula II is associated (i.e. coated in the pores and on the surface) with the silica particles prior to combining with the other components of the compositions of the invention. In one embodiment of the invention the weight percentage of the compound of Formula II to the silica particles is 20% ± 15%. In one embodiment of the invention the weight percentage of the compound of Formula II to the silica particles is 50% ± 10%. In one embodiment of the invention the weight percentage of the compound of Formula II to the silica particles is 45% ± 15%. In one embodiment of the invention the weight percentage of the compound of Formula II to the silica particles is 100% ± 20%. In one embodiment of the invention the weight percentage of the compound of Formula II to the silica particles is 85% ± 15%.

### Loading

The compound of Formula II can be loaded on the silica particles using any suitable method. For example the compound of Formula II can be loaded on the silica particles by:
a) spraying a solution of the compound (e.g. a solution of the compound in an alcohol solvent such as ethanol) onto the silica particles;
b) combining the compound of Formula II, a suitable solvent (e.g. a volatile solvent such as dichloromethane), and the silica particles; evaporating the solvent; and isolating the resulting solid material; or
c) combining the compound of Formula I and a suitable volatile solvent (e.g. a halogenated hydrocarbon such as dichloromethane), and the silica particles; adding an antisolvent (e.g. a highly non-polar solvent such as hexanes or heptane) and isolating the resulting solid material (as illustrated in Example 4).

The compound of Formula II can be combined with a suitable solvent and a plurality of silica particles to provide a mixture. Optionally, the compound of Formula II can be combined with the suitable solvent with concurrent mixing. Typically, the weight percentage of the compound of Formula II to the silica particles prior to combining is about is 50% ± 10%. In one embodiment of the invention the weight percentage of the compound of Formula II to the silica particles prior to combining is about 20% ± 10%. In another embodiment of the invention the weight percentage of the compound of Formula II to the silica particles prior to combining is about 30% ± 10%. Any solvent in which the compound of Formula II is soluble can be used. Typically, the solvent comprises a volatile organic solvent, such as, for example, a (C₁-C₆) alcohol (e.g. ethanol).

A compound of Formula II can also be loaded into a silica material by dissolving the compound in a suitable solvent to provide a solution comprising the compound of Formula II; adding silica particles to the solution to provide a mixture; optionally agitating or stirring the mixture; adding an antisolvent to the mixture; and isolating the solid mixture that comprises the compound of Formula II on the silica particles. Suitable solvents include organic solvents such as ketones (e.g. acetone), alcohols (e.g. ethanol) and halogenated hydrocarbons (e.g. dichloromethane). Suitable antisolvents include highly non-polar solvents (e.g. hexane or heptane). The final solid mixture can be isolated by any suitable separation technique (e.g. filtration).

One or more pharmaceutically acceptable excipients can be combined with the mixture to provide a second mixture. These pharmaceutically acceptable excipients can include fillers, binders, and disintegrants. In order to improve the processability of the mixture in the subsequent aqueous granulation process, it can be beneficial to select fillers and disintegrants that are compatible with this aqueous process. For example microcrystalline cellulose (filler) and croscarmellose sodium (disintegrant) were found to be particularly compatible with the subsequent aqueous granulation process. Hydroxypropyl cellulose (binder) was also found to be particularly compatible with the subsequent granulation process. In one embodiment of the invention the weight percentage of microcrystalline cellulose to the total weight of the second mixture is about 50% ± 20%. In one embodiment of the invention the weight percentage of hydroxypropyl cellulose to the total weight of the second mixture is 2% ± 1%. In one embodiment of the invention the weight percentage of croscarmellose sodium is 5% ± 2%. Following addition of the pharmaceutically acceptable excipients, the second mixture can be mixed, for example, using a mechanical mixer, such as a high shear granulator (Niro-Fielder, model PMA-25).

Water can be added to the second mixture to provide a wet granulate, which can subsequently be de-agglomerated, e.g. with a 20 mesh sieve. Drying, for example using a fluid bed dryer (Fluid Air, model 20), provides a dried material that comprises solid particles. In one embodiment the dried material has less than about 10.0% moisture content as determined by loss on drying (LOD). In another embodiment the dried material has less than about 5.0% moisture content as determined by loss on drying (LOD). In another embodiment the dried material has less than about 1.0% moisture content as determined by loss on drying (LOD). The size of these particles can be reduced, e.g. using a 40 mesh sieve or a suitable mill (Quadro CoMil, model 197/S) to provide a third mixture.

A suitable pharmaceutically acceptable lubricant/glidant (e.g. magnesium stearate, stearic acid, calcium stearate, zinc stearate, or pregelatinized starch) can be combined with the third mixture to provide a fourth mixture. In one embodiment the weight percentage of magnesium stearate to the total weight of the fourth mixture is 1% ± 0.5%.

In one embodiment, the invention provides a composition prepared by the methods described herein. The invention also provides a product prepared by any of the process steps described herein.

Formulations suitable for oral administration may be presented as discrete units such as tablets each containing a predetermined amount of the active ingredients; as a powder or granules.

A tablet can be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active agent or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient.

Tablets of the invention may contain one or more agents including sweetening agents, flavoring agents, coloring agents and preserving agents, in order to provide a palatable preparation. Tablets containing the active ingredients in admixture with non-toxic pharmaceutically acceptable excipients which are suitable for manufacture of tablets are acceptable. These excipients may be, for example, inert diluents, such as calcium or sodium carbonate, lactose, lactose monohydrate, croscarmellose sodium, povidone, calcium or sodium phosphate; granulating and disintegrating agents, such as maize starch, or alginic acid; binding agents, such as cellulose, microcrystalline cellulose, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

WO 2005/113508 provides certain specific crystalline forms of the compound of Formula I (in particular, see pages 12-62 therein). The specific crystalline forms are identified therein as Crystal Form II and Crystal Form III. Crystal form II has an X-ray powder diffraction pattern having characteristic diffraction peaks at diffraction angles 2θ(°) of 6.56, 13.20, 19.86, 20.84, 21.22, and 25.22 as measured by an X-ray powder diffractometer. Crystal form III has an X-ray powder diffraction pattern having characteristic diffraction peaks at diffraction angles 2θ(°) of 8.54, 14.02, 15.68, 17.06, 17.24, 24.16, and 25.74 as measured by an X-ray powder diffractometer. In one embodiment the compositions of the invention include a compound of formula I that is in Crystal Form II or Crystal Form III.

### Preparation of a Compound of Formula II and Preloading on Silica

A compound of Formula II or a salt thereof can be prepared by coupling an acid salt of formula X wherein M is a counterion with an amine of formula **IX** to form the corresponding amide of Formula II as described in WO 2008/103949 (for example, see page 254).

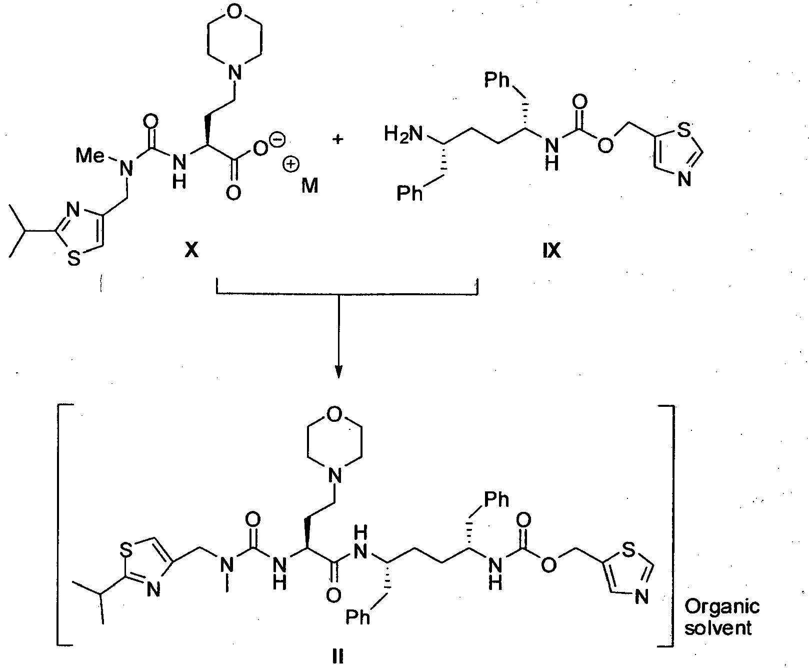

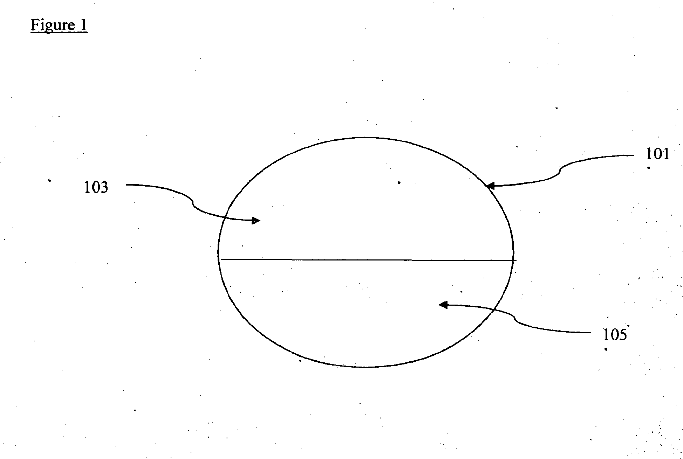

This amide forming reaction can be carried out under standard conditions. For example, it can be carried out in a suitable organic solvent (e.g. tetrahydrofuran or dichloromethane) in the presence of a suitable coupling agent (e.g. EDC•HCl and HOBt). Other suitable amide coupling reagents and conditions are known in the field. The reaction can typically be carried out at a temperature from about -30 °C to about 20 °C. The final reaction solution containing the compound of Formula II in dichloromethane (DCM) can be directly utilized in the processes illustrated in Figure 2 to provide representative compositions of the invention, or the dichloromethane solution of the compound can be combined with ethanol and the resulting mixture can be distilled to remove the dichloromethane, leaving a solution of the compound of Formula II in ethanol. This ethanol solution can be combined with the silicon dioxide particles and evaporated (as illustrated in the left column of Figure 2) to provide a composition comprising the compound of Formula II loaded on silicon dioxide particles. Alternatively, the dichloromethane solution of the compound can be combined with silicon dioxide particles, an antisolvent can be added, and the resulting mixture can be filtered and dried (as illustrated in the right column of Figure 2) to provide a composition comprising the compound of Formula II loaded
Figure 1 shows a cross-section of a tablet (101) of the invention. The tablet includes a first layer (103) that comprises a compound of Formula I and a compound of Formula II. The tablet also includes a second layer (105) that comprises a compound of Formula III and a salt of Formula IV.
Figure 2 illustrates processes that can be used to prepare a tablet comprising a compound of formula II.
Figure 3 illustrates a process for preparing a bilayer tablet of the invention. This process is further detailed in Example 1.

The invention will now be illustrated by the following Examples.

### Examples

### Example 1: Tablet Formation.

The manufacturing procedure for a fixed dose combination tablet containing the compounds of Formulas I, II, III and IV include the following steps: 1) fluid-bed granulation and drying of the compound of Formula I, 2) high-shear granulation and fluid-bed drying of the compound of Formula II or the compound of Formula II loaded on silica particles, 3) dry granulation of the compound of Formula III and dry granulation of the salt of Formula IV, 4) milling of the dry granulation of the compound of Formula III and milling of the dry granulation of the salt of Formula IV, 5) blending of the compound of Formula III and the salt of Formula IV, 6) blending of the compound of Formula I and the compound of Formula II, 7) bilayer compression with one layer consisting of the blend of the compounds of Formula I and Formula II and the other layer consisting of the blend of the compounds of Formula III and Formula IV to form a tablet, 8) coating of the tablet and 9) packaging of the coated tablet.

The in-process weight control for a bilayer tablet was superior compared to a trilayer tablet configuration. Bilayer weight control for the layer containing the compounds of Formula I and Formula II was between 100.2% and 100.8% of the mean target layer weight. Mean weights for the total tablet was between 99.5% and 100.7% of the mean target tablet weight. The relative standard deviation (RSD) value for the layer containing the compounds of Formula I and Formula II was between 1.4% and 2.2%, while the RSD for the total tablet was between 0.7% and 1.2%. These low RSD values indicate very low weight variability during the bilayer tablet compression process. The friability at the start and end of the compression process was 0.0%. No chipped, capped, or broken tablets were observed during bilayer compression.

Significant material property and compressibility differences between layer 1 and layer 2 presented the risk of layer separation during the tablet compression process using the process of preloading Formula II on a solid carrier. However, the resulting tablets were intact with no defects and withstood the aggressive film-coating process.

As described above, an intermediate mixture that is useful for preparing a layer of a tablet of the invention is a composition that comprises the compound of formula I and the compound of formula II. Accordingly, in one embodiment, the invention provides a composition comprising the compound of formula I and the compound of formula II. The compounds of formula I and II may be present in any of the amounts or ratios described herein that are useful for preparing tablets of the invention. For example, the following tables illustrate representative components and weight ratios for intermediate compositions that can be used to prepare tablets of the invention.

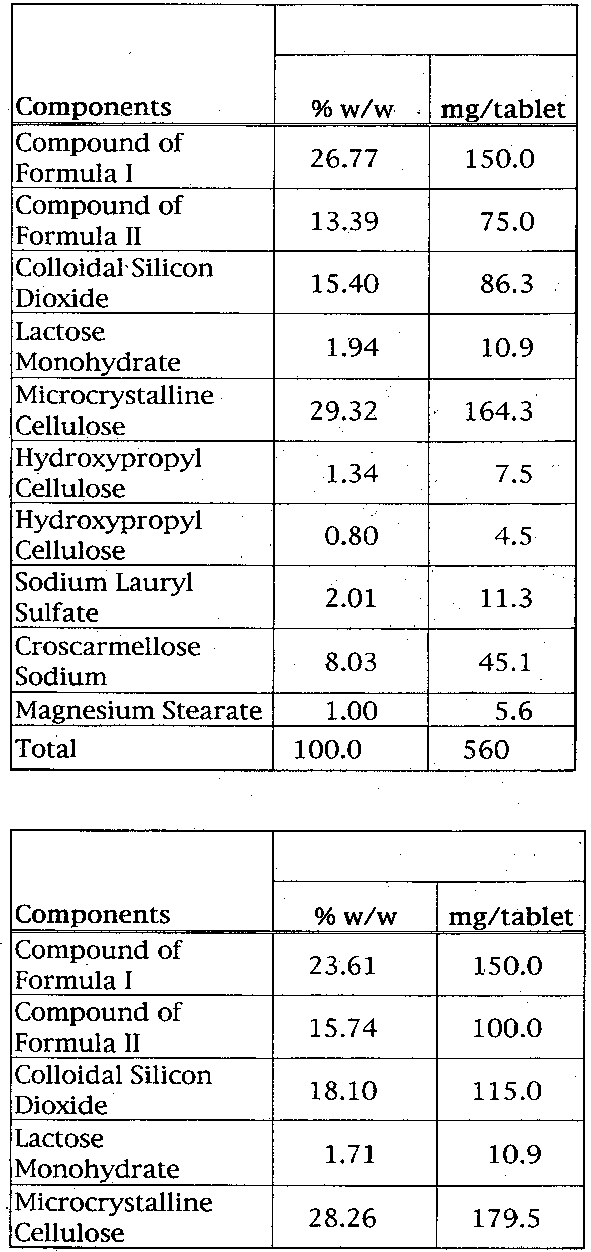

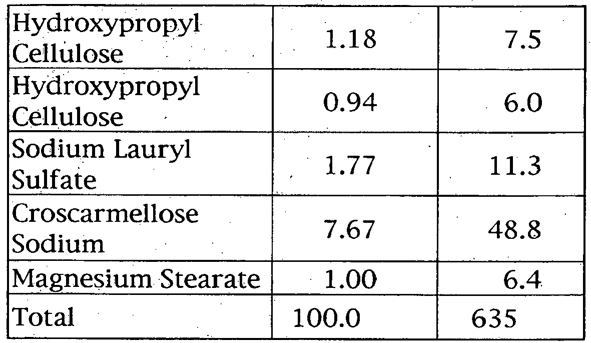

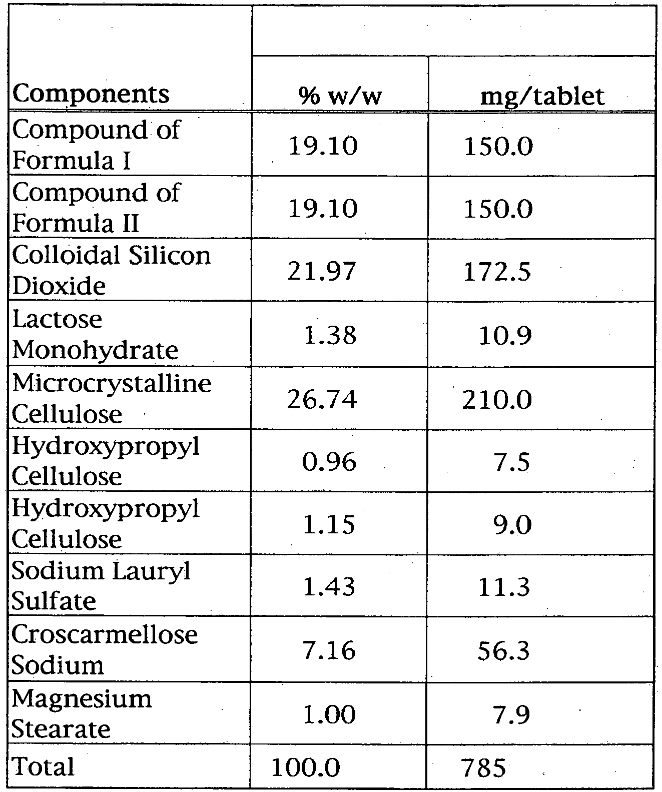

Attempts were also made to manufacture the tablet as a trilayer. During trilayer compression, the tablet was manufactured with a layer of the compound of Formula I, a second layer containing the compound of Formula II and a third layer containing a blend of the compound of Formula III and the salt of Formula IV. The initial attempt was to apply the compound of Formula II as the first layer, followed by the compound of Formula I on the second layer, and lastly the third layer of a blend of the compound of Formula III and the salt of Formula IV. The weight of the layer containing the compound of Formula II was too small, which resulted in a very thin tablet layer that caused a collision of the upper and lower punches during the compression process. The weight of the layer containing the compound of Formula I was similar to the weight of the layer containing the compound of Formula II so punch collision would prevent the reverse configuration. The weight of the blend containing the compounds of Formula III and IV was large enough to present a layer thickness that prevented punch collision during the compression process. This layer was followed by a second layer containing the compound of Formula I and a third layer containing the compound of Formula II.

Severe tablet defects were observed during the tablet compression process involving the three layer process. There was also difficultly controlling the tablet weight with this three layer configuration as well. In addition, the second and third layers separated from the tablet core. An intact tablet, free of defects could not be produced for the trilayer tablet configuration.

Combining all of the compounds of Formula I, II, III, and IV in a mixture to produce a mono layer tablet compromises the chemical stability of Formula IV. Additionally, improper combinations of the compounds of Formula I, II, III, or IV in a bilayer tablet will also compromise the chemical stability of Formula IV. For example, Formula IV in the presence of silicon dioxide from the Formula II formulation will decompose Formula IV; and Formula IV in the presence of sodium lauryl sulphate from the Formula I formulation will decompose Formula IV.

### Example 2

The following table illustrates representative compositions of the invention that are useful in preparing tablets of the invention.

| | **Compound of Formula II 75 mg Formulation** | | **Compound of Formula II 100 mg Formulation** | |
|---|---|---|---|---|
| **Components** | **% w/w** | **mg/tablet** | **% w/w** | **mg/table t** |
| Compound of Formula III | 16.5 | 200.0 | 15.5 | 200.0 |
| Salt of Formula IV | 24.7 | 300.0 | 23.3 | 300.0 |
| Compound of Formula I | 12.4 | 150.0 | 11.7 | 150.0 |
| Compound of Formula II | 6.2 | 75.0 | 7.8 | 100.0 |
| Colloidal Silicon Dioxide | 7.1 | 86.3 | 8.9 | 115.0 |
| Lactose Monohydrate | 0.9 | 10.9 | 0.8 | 10.9 |
| Microcrystalline Cellulose | 20.9 | 253.8 | 20.9 | 269.0 |
| Hydroxypropyl Cellulose | 0.6 | 7.5 | 0.6 | 7.5 |
| Hydroxypropyl Cellulose | 0.4 | 4.5 | 0.5 | 6.0 |
| Sodium Lauryl Sulfate | 0.9 | 11.3 | 0.9 | 11.3 |
| Croscarmellose Sodium | 7.7 | 93.1 | 7.5 | 96.8 |
| Magnesium Stearate | 1.7 | 20.1 | 1.6 | 20.9 |
| Total | 100 | 1212 | 100 | 1287 |

### Example 3

The following table illustrates a representative composition of the invention that is useful in preparing tablets of the invention.

| | **Compound of Formula II 150 mg Formulation** | |
|---|---|---|
| **Components** | **% w/w** | **mg/tablet** |
| Compound of Formula III | 13.9 | 200.0 |
| Salt of Formula IV | 20.9 | 300.0 |
| Compound of Formula I | 10.4 | 150.0 |
| Compound of Formula II | 10.4 | 150.0 |
| Colloidal Silicon Dioxide | 12.0 | 172.5 |
| Lactose Monohydrate | 0.8 | 10.9 |
| Microcrystalline Cellulose | 20.8 | 299.5 |
| Hydroxypropyl Cellulose | 0.5 | 7.5 |
| Hydroxypropyl Cellulose | 0.6 | 9.0 |
| Sodium Lauryl Sulfate | 0.8 | 11.3 |
| Croscarmellose Sodium | 7.3 | 104.3 |
| Magnesium Stearate | 1.6 | 22.4 |
| Total | 100 | 1437 |

### Example 4

The following table illustrates a representative composition of the invention that is useful in preparing tablets of the invention.

| | **Compound of Formula II 150 mg Formulation** | |
|---|---|---|
| **Components** | **% w/w** | **mg/tablet** |
| Compound of Formula III | 14.8 | 200.0 |
| Salt of Formula IV | 22.2 | 300.0 |
| Compound of Formula I | 11.1 | 150.0 |
| Compound of Formula II | 11.1 | 150.0 |
| Lactose Monohydrate | 0.8 | 10.9 |
| Microcrystalline Cellulose | 19.5 | 267.1 |
| Colloidal Silicon Dioxide | 11.1 | 150.0 |
| Croscarmellose Sodium | 6.1 | 81.75 |
| Hydroxypropyl Cellulose | 0.6 | 7.5 |
| Sodium Lauryl Sulfate | 0.8 | 11.25 |
| Magnesium Stearate | 1.6 | 21.5 |
| Tablet Core Weight | 100 | 1350 |

### Example 5.

A pharmacokinetic dose-ranging study involving the compound of Formula II was conducted. Doses for the compound of Formula II for the dose-ranging study included 50 mg, 100 mg and 200 mg. Pharmacokinetic data for the compound of Formula II from the dose-ranging study for the 100 mg dose is provided in the table below.

| PK Data for the compound of Formula II | |
|---|---|
| (dose-ranging study; 100 mg dose; n=11) | |
| AUCₜₐᵤ (ng.hr/mL) | 3440 (34.3) |
| Cₘₐₓ (ng/mL) | 563 (30.7) |
| T_{1/2} (h) | 3.12 (2.55, 3.36) |
| CL/F (L/h) | 33.2 (43.6) |

| | |
|---|---|
| Q1 = first quartile; Q3 = third quartile Data presented as arithmetic mean (%CV); T_{½} presented as median (Q1, Q3); %CV, percent coefficient of variation | |

A pharmacokinetic study involving two fixed-dose combinations of the compounds of Formula I/ Formula II/ Formula III/ Formula IV was also conducted. Doses for the fixed-dose combination of the compounds of Formula I/ Formula II/ Formula III/ Formula IV were 150 mg/100 mg/ 200 mg/300 mg (Dose A) and 150 mg/150 mg/ 200 mg/300 mg (Dose B) respectively. Phamacokinetic data for the compound of Formula II from the study involving the two fixed dose combinations (Dose A and Dose B) is provided in the following table.

| PK Data for the compound of Formula II | | |
|---|---|---|
| (fixed dose combination study) | | |
| Mean (CV%) PK | Dose A (n = 43) | Dose B (n = 42) |
| AUCₜₐᵤ (ng.hr/mL) | 515 (31.7) | 10400 (35.1) |
| Cmax (ng/mL) | 855 (27.6) | 1570(29.7) |
| Cₜₐᵤ (ng/mL) | 10.8(83.6) | 23.3 (103) |

The overall exposure, as measured by area under the curve (AUC), was 1.5 to 2-fold higher for the compound of Formula II in the fixed-dose combination study (Dose A) versus the exposure as measured by area under the curve (AUC) for the dose-ranging study involving the 100 mg dose of the compound of Formula II. Due to this increased exposure to the compound of Formula II, either less of the compound of Formula I is required to provide an equivalent antiviral effect, or the same amount of the compound of Formula I will provide an increased antiviral effect when the compound of Formula I is administered with the compound of Formula II. This unexpected increased exposure to the compound of Formula II, also suggests that less compound of Formula II is required to achieve effective exposure to the compound of Formula II.

## Claims

1. A tablet comprising a first layer and a second layer wherein;
a) the first layer comprises:
a compound of Formula I: 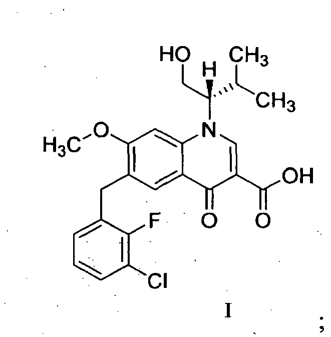 and
a compound of Formula II: 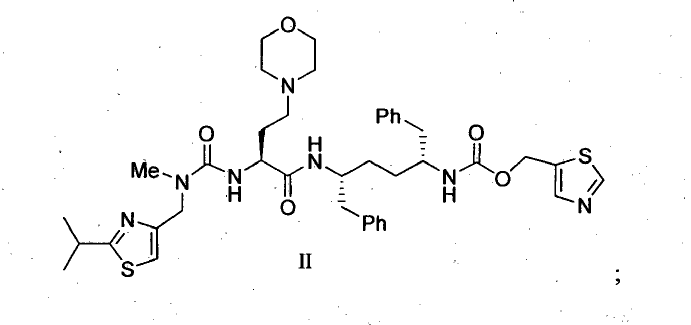
and optionally a pharmaceutically acceptable carrier; and
b) the second layer comprises:
a compound of Formula III: 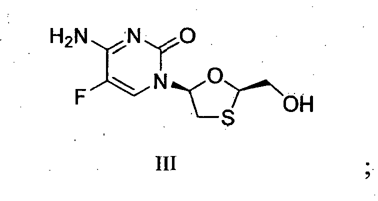 and
a salt of Formula IV: 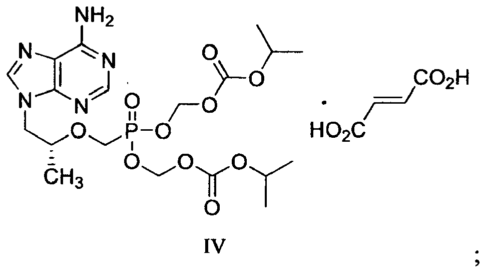
and optionally a pharmaceutically acceptable carrier.

2. The tablet of claim 1 wherein the first layer is in contact with the second layer.

3. The tablet of any one of claims 1-2 wherein the weight ratio of the compound of Formula I to the compound of Formula II is 1.0 ± 0.5.

4. The tablet of any one claims 1-3 wherein the first layer further comprises a plurality of silica particles.

5. The tablet of any one claims 1-3 wherein the compound of Formula II is associated with silica particles.

6. The tablet of any one of claims 1-5 that comprises 150 mg ± 10% of the compound of Formula I; 150 mg ± 10% of the compound of Formula II; 200 mg ± 10% of the compound of Formula III; and 300 mg ± 10% of the compound of Formula IV.

7. The tablet of claim 6 that comprises 150 mg ± 5% of the compound of Formula I.

8. The tablet of claim 6 that comprises 150 mg ± 2% of the compound of Formula I.

9. The tablet of any one of claims 6-8 that comprises 150 mg ± 5% of the compound of Formula II.

10. The tablet of any one of claims 6-8 that comprises 150 mg ± 2% of the compound of Formula II.

11. The tablet of any one of claims 6-10 that comprises 200 mg ± 5% of the compound of Formula III.

12. The tablet of any one of claims 6-10 that comprises 200 mg ± 2% of the compound of Formula III.

13. The tablet of any one of claims 6-12 that comprises 300 mg ± 5% of the salt of Formula IV.

14. The tablet of any one of claims 6-12 that comprises 300 mg ± 2% of the salt of Formula IV.

15. The tablet of any one of claims 1-14 further comprising a pharmaceutically acceptable carrier.

16. The tablet of any one of claims 1-15 which is formulated in unit dose form.

17. The tablet of any one of claims 1-16 which is formulated for once a day dosing.

18. A method for preparing a tablet as described in claim 1 comprising: compressing a composition that comprises the compound of formula I and the compound of formula II to provide a first pressed layer; adding the compound of formula III and the salt of formula IV to the first pressed layer; and compressing to provide the tablet.

19. A tablet as described in claim 1 that consists of the following:
| Components | Compound of Formula II 150 mg Formulation | |
|---|---|---|
| | % w/w | mg/tablet |
| Compound of Formula III | 13.9 | 200.0 |
| Salt of Formula IV | 20.9 | 300.0 |
| Compound of Formula I | 10.4 | 150.0 |
| Compound of Formula II | 10.4 | 150.0 |
| Colloidal Silicon Dioxide | 12.0 | 172.5 |
| Lactose Monohydrate | 0.8 | 10.9 |
| Microcrystalline Cellulose | 20.8 | 299.5 |
| Hydroxypropyl Cellulose | 0.5 | 7.5 |
| Hydroxypropyl Cellulose | 0.6 | 9.0 |
| Sodium Lauryl Sulfate | 0.8 | 11.3 |
| Croscarmellose Sodium | 7.3 | 104.3 |
| Magnesium Stearate | 1.6 | 22.4 |
| Total | 100 | 1437. |

20. A tablet prepared as described in claim 18.

## Patentansprüche

1. Tablette, umfassend eine erste Schicht und eine zweite Schicht, wobei
a) die erste Schicht:
eine Verbindung der Formel I: 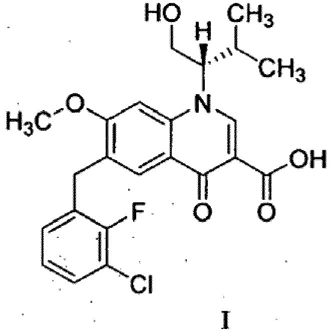 und
eine Verbindung der Formel II: 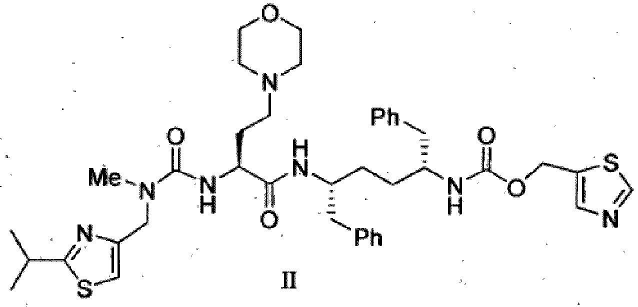
und gegebenenfalls einen pharmazeutisch unbedenklichen Träger umfasst und
b) die zweite Schicht:
eine Verbindung der Formel III: 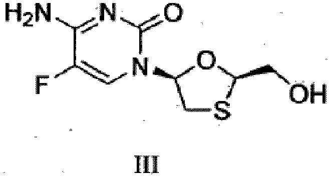 und
ein Salz der Formel IV: 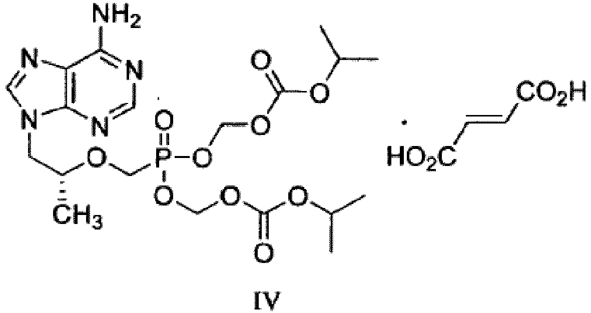
und gegebenenfalls einen pharmazeutisch unbedenklichen Träger umfasst.

2. Tablette nach Anspruch 1, wobei sich die erste Schicht in Kontakt mit der zweiten Schicht befindet.

3. Tablette nach einem der Ansprüche 1-2, wobei das Gewichtsverhältnis der Verbindung der Formel I zur Verbindung der Formel II 1,0 ± 0,5 beträgt.

4. Tablette nach einem der Ansprüche 1-3, wobei die erste Schicht weiterhin mehrere Siliziumdioxidpartikel umfasst.

5. Tablette nach einem der Ansprüche 1-3, wobei die Verbindung der Formel II mit Siliziumdioxidpartikeln assoziiert ist.

6. Tablette nach einem der Ansprüche 1-5, umfassend 150 mg ± 10% der Verbindung der Formel I, 150 mg ± 10% der Verbindung der Formel II, 200 mg ± 10% der Verbindung der Formel III und 300 mg ± 10% der Verbindung der Formel IV.

7. Tablette nach Anspruch 6, umfassend 150 mg ± 5% der Verbindung der Formel I.

8. Tablette nach Anspruch 6, umfassend 150 mg ± 2% der Verbindung der Formel I.

9. Tablette nach einem der Ansprüche 6-8, umfassend 150 mg ± 5% der Verbindung der Formel II.

10. Tablette nach einem der Ansprüche 6-8, umfassend 150 mg ± 2% der Verbindung der Formel II.

11. Tablette nach einem der Ansprüche 6-10, umfassend 200 mg ± 5% der Verbindung der Formel III.

12. Tablette nach einem der Ansprüche 6-10, umfassend 200 mg ± 2% der Verbindung der Formel III.

13. Tablette nach einem der Ansprüche 6-12, umfassend 300 mg ± 5% des Salzes der Formel IV.

14. Tablette nach einem der Ansprüche 6-12, umfassend 300 mg ± 2% des Salzes der Formel IV.

15. Tablette nach einem der Ansprüche 1-14, weiterhin umfassend einen pharmazeutisch unbedenklichen Träger.

16. Tablette nach einem der Ansprüche 1-15, formuliert in Einheitsdosisform.

17. Tablette nach einem der Ansprüche 1-16, formuliert für eine einmal tägliche Verabreichung.

18. Verfahren zur Herstellung einer wie in Anspruch 1 beschriebenen Tablette, bei dem man: eine die Verbindung der Formel I und die Verbindung der Formel II umfassende Zusammensetzung zu einer ersten verpressten Schicht komprimiert, die erste verpressete Schicht mit der Verbindung der Formel III und dem Salz der Formel IV versetzt und unter Bereitstellung der Tablette komprimiert.

19. Tablette nach Anspruch 1, welche aus den folgenden Bestandteilen besteht:
| Komponenten | Verbindung der Formel II 150 mg Formulierung | |
|---|---|---|
| | Gew.-% | mg/Tablette |
| Verbindung der Formel III | 13,9 | 200,0 |
| Salz der Formel IV | 20,9 | 300,0 |
| Verbindung der Formel I | 10,4 | 150,0 |
| Verbindung der Formel II | 10,4 | 150,0 |
| Kolloidales Siliziumdioxid | 12,0 | 172,5 |
| Lactosemonohydrat | 0, 8 | 10, 9 |
| Mikrokristalline Cellulose | 20,8 | 299,5 |
| Hydroxypropylcellulose | 0,5 | 7,5 |
| Hydroxypropylcellulose | 0,6 | 9,0 |
| Natriumlaurylsulfat | 0,8 | 11,3 |
| Croscarmellose-Natrium | 7,3 | 104,3 |
| Magnesiumstearat | 1,6 | 22,4 |
| Gesamt | 100 | 1437 |

20. Tablette, hergestellt wie in Anspruch 18 beschrieben.

## Revendications

1. Comprimé comprenant une première couche et une deuxième couche, où :
a) la première couche comprend :
un composé de Formule I : 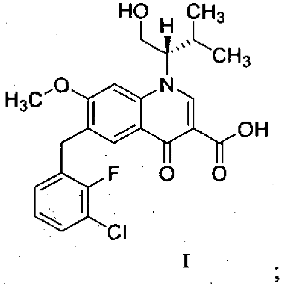
un composé de Formule II : 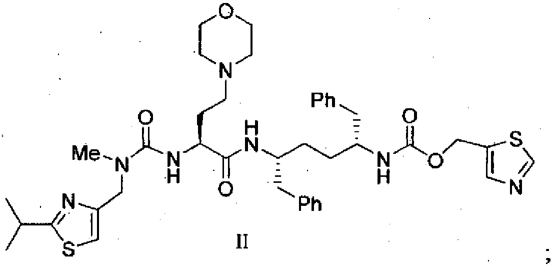
et éventuellement un vecteur pharmaceutiquement acceptable ; et
b) la deuxième couche comprend :
un composé de Formule III : 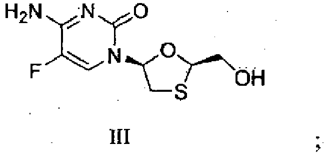 et
un sel de Formule IV : 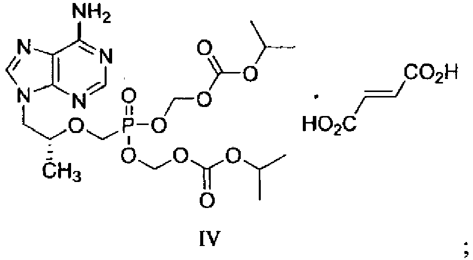
et éventuellement un vecteur pharmaceutiquement acceptable.

2. Comprimé selon la revendication 1, où la première couche est en contact avec la deuxième couche.

3. Comprimé selon l'une quelconque des revendications 1 à 2, où le rapport massique du composé de Formule I sur le composé de Formule II est de 1,0 ± 0,5.

4. Comprimé selon l'une quelconque des revendications 1 à 3, où la première couche comprend en outre une multitude de particules de silice.

5. Comprimé selon l'une quelconque des revendications 1 à 3, où le composé de Formule II est associé à des particules de silice.

6. Comprimé selon l'une quelconque des revendications 1 à 5, qui comprend 150 mg ± 10 % du composé de Formule I ; 150 mg ± 10 % du composé de Formule II ; 200 mg ± 10 % du composé de Formule III ; et 300 mg ± 10 % du composé de Formule IV.

7. Comprimé selon la revendication 6, qui comprend 150 mg ± 5 % du composé de Formule I.

8. Comprimé selon la revendication 6, qui comprend 150 mg ± 2 % du composé de Formule I.

9. Comprimé selon l'une quelconque des revendications 6 à 8, qui comprend 150 mg ± 5 % du composé de Formule II.

10. Comprimé selon l'une quelconque des revendications 6 à 8, qui comprend 150 mg ± 2 % du composé de Formule II.

11. Comprimé selon l'une quelconque des revendications 6 à 10, qui comprend 200 mg ± 5 % du composé de Formule III.

12. Comprimé selon l'une quelconque des revendications 6 à 10, qui comprend 200 mg ± 2 % du composé de Formule III.

13. Comprimé selon l'une quelconque des revendications 6 à 12, qui comprend 300 mg ± 5 % du sel de Formule IV.

14. Comprimé selon l'une quelconque des revendications 6 à 12, qui comprend 300 mg ± 2 % du sel de Formule IV.

15. Comprimé selon l'une quelconque des revendications 1 à 14, comprenant en outre un vecteur pharmaceutiquement acceptable.

16. Comprimé selon l'une quelconque des revendications 1 à 15, qui est formulé sous la forme d'une dose unitaire.

17. Comprimé selon l'une quelconque des revendications 1 à 16, qui est formulé pour administration une fois par jour.

18. Procédé d'élaboration d'un comprimé selon la revendication 1 comprenant : la compression d'une composition qui comprend le composé de formule I et le composé de formule II pour obtenir une première couche pressée ; l'ajout du composé de formule III et du sel de formule IV à la première couche pressée ; et la compression pour obtenir le comprimé.

19. Comprimé selon la revendication 1, qui est constitué des composants suivants :
| Composants | Composé de Formule II, formule de 150 mg | |
|---|---|---|
| | % massique | mg/comprimé |
| Composé de Formule III | 13,9 | 200,0 |
| Sel de Formule IV | 20, 9 | 300,0 |
| Composé de Formule I | 10,4 | 150,0 |
| Composé de Formule II | 10,4 | 150,0 |
| Dioxyde de silicium colloïdal | 12,0 | 172,5 |
| Lactose monohydraté | 0, 8 | 10, 9 |
| Cellulose microcristalline | 20,8 | 299,5 |
| Hydroxypropylcellulose | 0,5 | 7,5 |
| Hydroxypropylcellulose | 0, 6 | 9, 0 |
| Laurylsulfate de sodium | 0,8 | 11,3 |
| Croscarmellose sodium | 7,3 | 104,3 |
| Stéarate de magnésium | 1,6 | 22,4 |
| Total | 100 | 1437 |

20. Comprimé élaboré selon la revendication 18.
